Europäisches Patentamt

European Patent Office

Office européen des brevets

(11) Publication number: **0 151 697**
**B1**

(12) **EUROPEAN PATENT SPECIFICATION**

(45) Date of publication of patent specification: **17.11.88**

(21) Application number: **84112895.2**

(22) Date of filing: **25.10.84**

(51) Int. Cl.⁴: **C 07 C 17/38**

(54) Method for purifying a mixture of perfluorocarbons.

| | |
|---|---|
| (30) Priority: **31.10.83 US 547314**<br>**17.09.84 US 650679** | (73) Proprietor: **ADAMANTECH, INC.**<br>**Second and Green Streets P.O. Box 1195**<br>**Marcus Hook Pennsylvania 19061 (US)** |
| (43) Date of publication of application:<br>**21.08.85 Bulletin 85/34** | (72) Inventor: **Bierl, Thomas W.**<br>**2326 McNary Boulevard**<br>**Pittsburgh Pennsylvania (US)** |
| (45) Publication of the grant of the patent:<br>**17.11.88 Bulletin 88/46** | (74) Representative: **Baillie, Iain Cameron et al**<br>**c/o Ladas & Parry Isartorplatz 5**<br>**D-8000 München 2 (DE)** |
| (84) Designated Contracting States:<br>**AT BE CH DE FR GB IT LI LU NL SE** | |
| (56) References cited:<br>**EP-A-0 099 652**<br>**US-A-2 738 371**<br>**US-A-2 999 885**<br>**US-A-3 696 156**<br><br>**Analytical Chem. 26 (1954), 523-525** | |

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to the purification of fluorocarbon compounds to remove hydrogen-containing materials and/or to render the fluorocarbons less toxic to living organisms.

Biological applications of fluorocarbon compounds have expanded in recent years, therefore requiring careful attention to purification of the compound to provide pharmaceutical grade material. Typical of such applications are the blood substitute and perfusion media uses of perfluorinated cyclic hydrocarbons described in U.S. patents 3,911,138—Clark and 4,105,798—Moore and Clark. The latter patent describes the purification of a perfluoro polycyclic hydrocarbon mixture by refluxing with 10% aqueous potassium hydroxide (KOH). It is also known that fluorocarons may be purified prior to biological evaluations by refluxing with 4N aqueous KOH for several hours to remove partially fluorinated, hydrogen-containing by-products.

However, even with several treatments with farily concentrated KOH solutions (about 30 wt.%), the known treatments have reduced the hydrogen content only about 30—50%, e.g., in one type of fluorocarbon from about 1000 ppm in the starting material to about 500—700 ppm in the product, and in another type from about 100 ppm in the starting material to about 50 ppm in the product. (In this specification, ppm of hydrogen means the weight of hydrogen atoms relative to the total weight of the fluorocarbon compounds determined by infrared spectroscopy). Since it is now generally believed that for biological applications, especially for treatment of human medical conditions, the hydrogen content of the fluorocarbons shoudl be less than about 25 ppm, preferably less than about 5 ppm, it is apparent that the conventional KOH purification procedure is inadequate, even though the conventional procedure, when measured by some toxicity test procedures, appears to substantially reduce or even eliminate toxicity in the purified product. The reason for this inadequacy is that substantial testing of perfluorocarbons in humans has not yet occurred. Therefore and also because toxicity is actually a function of both the nature of the species which contains hydrogen, as well as the hydrogent content, it is not known for sure what level of these hydrogen containing species can be regarded as non-toxic. Also, it is not known for sure whether hydrogen containing species are toxic or, if not toxic, whether it reacts in the body to form something which is toxic. In view of the lack of knowledge about human toxicity, and the tremendous risks associated with erroneous judgments in respect thereof, the best solution is simply to remove as much of the hydrogen as possible and then evaluate the product in the basis of the amount of any residual hydrogen present and on toxicity tests of the product. In this regard the present process is much more effective in producing a less toxic product than the existing process.

"Purification" or similar term as used in this specification means elimination or substantial reduction of (1) acute toxicity as determined by in vitro tests such as the L-cell test or in vivo $LD_{50}$ test, and/or (2) hydrogen content.

In U.S. patent 3,696,156—Weeks, KOH or other base, deposited on an alumina carrier, is disclosed for treatment of lower molecular weight fluoroperhalocarbons, containing two to six carbons atoms. This patent discloses treating saturated fluoroperhalocarbons with an agent prepared by dissolving alkali metal hydroxide in just sufficient amount of water to wet the surface of alumina, driving off gross water by gentle heating and then heating from about 250°C to about 400°C in a stream of nitrogen. The purpose of the treatment is to remove toxic unsaturated impurities such as octafluorobutenes, so that the fluoroperhalocarbon product will be sufficiently non-toxic for use as an aerosol propellant. There is no disclosure that the treating agent would be effective to solve the toxicity problems connected with hydrogen-containing compounds contained as impurities in higher molecular weight fluorocarbons which are useful in blood substitutes.

Prior copending European Patent Publication 99652 (which designates all member states except Austria, Italy and Switzerland) describes a means for treating perfluoro compounds by refluxing the same with equal volumes of 70% potassium hydroxide and diisobutylamine.

An article by Grafstein entitled "Detection, Estimation and Removal of Impurities in Fluorocarbon Liquids" Analytical Chemistry 26 523 (1954) discloses the use of potassium hydroxide pellets to remove hydrocarbon-containing impurities from perfluorocarbons.

Summary of the invention

It has now been found that perfluorocarbons can be purified to levels required for biological uses by reaction with a strongly basic and strongly nucleophilic compound wherein the compound contains no more than 30 wt.% water, as opposed to the 5—30 wt.% KOH solutions of prior art purification techniques for such perfluorocarbons followed by treatment with an organic carbonyl compound and recovering the fluorocarbon phase. In the purification process the hydrogen-containing molecules and/or other impurities are reacted with the basic compound and the reaction products are separated from the perfluorocarbon molecules. "Perfluorocarbons", as the term is used herein, refers to fluorinated organic compounds containing no hydrogen in the molecule. "Fluorocarbons" on the other hand include, in addition to perfluorocarbons, fluorinated organic compounds containing hydrogen in the molecule. The expressions perfluoro and F are synonomous.

A major benefit of the purification technique of the invention is the ability to achieve a higher purity with essentially a single reaction with the base (in the case of fluorocarbons containing about 100—200 ppm hydrogen) than can be obtained with the multiple reactions and extractions employed in prior processes. Fewer reactions also has the concomitant advantages of less opportunity for loss of desired product and lower production cost. The novel reaction will also facilitate obtaining even higher levels of purity than in prior purification processes, by adjustment of treatment conditions, such as temperature, work-up procedures, and in the case of fluorocarbons containing higher amounts of hydrogen, e.g., over 200 ppm, one or more repetitions of the reaction. These and other aspects will be detailed in the description which follows.

Detailed description

In accordance with the invention, perfluorocarbons are purified by contact with a strongly basic alkali metal and/or alkaline earth metal compound (groups I and II respectively or The Periodic Table). Suitable bases are those which have logarithmic basicity constants (pKb) of at least 12, preferably 13 to 14. It is believed that the bases have strong nucleophilic activity, sufficient for successful attack on the hydrogen containing molecule as described below. Compounds of lithium, sodium, potassium, magnesium, calcium, barium and strontium are preferred. Suitable compounds include hydroxides, oxides (both inorganic and organic metal oxides), as well as other compounds having adequate basicity.

Representative metal hydroxides are sodium hydroxide, potassium hydroxide, lithium hydroxide, magnesium hydroxide, calcium hydroxide, strontium hydroxide and barium hydroxide. Potassium hydroxide is particularly preferred. The organic metal oxides may be represented by the formula $M(OR)_x$ where M is the metal, R is alkyl or aryl (including aralkyl and alkaryl), and x is the valence of the metal M (1 or 2). The organic metal oxides can contain from 1 to about 20 carbon atoms in each R group, preferably 4—10. The higher the number of carbon atoms the easier the subsequent extraction of reaction residues becomes, as is shown hereinafter. The R groups independently may be straight or branched chain, alicyclic or aromatic, including combinations of aliphatic, alicyclic and aromatic groups, and may carry substituents containing hetero atoms (such as halogen) provided such substituents do not detract from the essential properties of the compounds in the present invention. Typical R groups are $C_1$—$C_8$ alkyl such as methyl, ethyl and propyl; cycloalkyl such as cyclohexyl and cycloheptyl; and aryl groups such as phenyl, naphthyl, benzyl and phenethyl, and groups of the foregoing types substituted with halogen, such as fluoro, chlor and bromo, or nitro. A variety of metal aryloxides and alkoxides are known which are useful in the process. Some of these are described and listed in the technical literature, such as Kirk-Othmer, Encylopedia of Chemical Technology, Second Edition (1963), 1, 839, and include the methoxides, ethoxides and phenoxides of lithium, sodium, potassium and magnesium.

The strongly basic compounds employed according to the invention may contain water, in amount not exceeding about 30 wt.%. In the case of metal hydroxides, the amount may be such as to manifest a slightly tacky or pasty condition, but not a syrupy or other liquid state. In the case of the metal oxides, the amount of water is generally less than the amount, e.g about 5 wt.%, which will substantially hydrolyze the oxide. The compound may be used in pellet form, but other forms are useful such as powders or flakes. The compound may also be used by dispersing or dissolving the same in a non-aqueous solvent (such as an alcohol, ether or ketone) to improve contact between the fluorocarbon and base phases of the reaction mixture provided, of course, that the solvent does not substantially diminish the strongly basic and strongly nucleophilic character of the base, e.g., by complex formation or other interaction between solvent, base and/or fluorocarbon.

In connection with the strong basicity and nucleophilicity of the bases, it is belived that the excellent purification achieved by the process of the invention is probably obtained by the following stepwise mechanism, although the invention is not to be limited by any theory. In the first step the fluorocarbon is attacked by the base which has the ability to remove even the most strongly bonded hydrogen atoms in a molecule which is otherwise highly fluorinated. The removal of the hydrogen results in a negatively charged fluorinated ion which is unstable. Consequently, it undergoes a molecular rearrangement with the resultant elimination of a fluoride ion and the stabilization of the molecule as a perfluoro-olefin. In the second step, the hydroxyl or alkoxide ion of the base, acting as a strong nucleophile, attacks the olefin at the double bond, adding to the molecule a hydroxyl or other functional group and again changing the molecule into an unstable negative ion which rearranges to form another olefin. In the special case where the attacking base is a metal hydroxide and the hydroxyl functional group is on a carbon which also has fluorine bonded to it, hydrogen fluoride is most probably eliminated forming a ketone. In Step 3 the olefin formed in Step 2 is again attacked by hydroxyl ion and the process continues as described above until the fluorine is nearly completely stripped from the carbon skeleton. The resulting species is highly oxygenated, resulting in its being partitioned into the water phase and out of the fluorocarbon phase in the subsequent extraction step, leaving behind the purified fluorocarbon.

Compounds which are not as strongly basic as those of the invention and not used in a substantially dry state are not capable of reacting with all of the different possible hydrogen positions in the molecule. If the hydrogen is not removed, the chain reaction is not initiated and the purification is not achieved. Moreover, if the anion of the metal hydroxide or organic metal oxide is not a strong nucleophile, it will not propagate the chief reaction by attacking the olefin formed during the first rearrangement. Without this nucleophilic addition to the double bond, the perfluoro olefin is not removable from the remaining

EP 0 151 697 B1

saturated perfluorocarbon. It is this addition of the hydroxyl, alkoxide or aryloxide ion of the strong base which allows the final reaction product to be selectively extracted by either water or organic solvent.

The fluorocarbon feed material to the process of the invention is reaction product from the fluorination of carbon compounds, normally hydrocarbons, which product contains perfluorinated molecules not subject to attack by strongly basic and strongly nucleophilic compounds and some other fluorinated molecules or other substances which contain hydrogen and are therefore subject to such attack. They are impure perfluorocarbons, but are sometimes referred to as perfluorocarbons even though the latter description is not strictly accurate if there is any hydrogen present. The carbon compounds are aliphatic (cyclic or acyclic) hydrocarbons and/or hereto atom containing aliphatic carbon compounds, which, except for the residual hydrogen and/or toxic components, have been fully flourinated. The compounds or mixtures may be in the vapor or liquid state at ambient temperatures and pressures. Solid compounds are purifiable in accordance with the invention by dispersion or dissolution in a liquid medium, by melting or by vaporization.

The invention has special significance for the purification of the blood substitute perfluorinated cyclic hydrocarbons described in U.S. Patents 3,911,138 and 4,105,798, (both incorporated herein by reference) but the technique is also applicable to other fluorinated compounds such as are described in U.S. Patents 3,823,091, 3,962,439 and 4,325,972.

Perfluorocarbons for treatment according to the invention, are those which contain 7 to 18 carbon atoms in the molecule, and more preferably those which contain 9 to 18 carbon atoms, because such perfluorocarbons are suitable for use in biological applications. Representative compounds are the perfluorinated derivatives of chemically inert monocyclic compounds such as isopropylcyclopentane or isopropylcyclohexane, and $C_9$—$C_{18}$ polycyclic compounds such as bicyclononanes (e.g., bicyclo [3.3.1] nonane, and 2,6 - dimethylbicyclo [3.3.1] nonane, 3 - methylbicyclo [3.3.1] nonane and trimethylbicyclo [3.3.1] nonane); adamantane and alkyl ($C_1$—$C_6$) adamantanes such as methyl and dimethyladamantane, ethyl and diethyladamantane, trimethyladamantane, ethylmethyladamantane, ethyldimethyladamantane and triethyladamantane; methyldiadamantane and trimethyldiadamantane; methyl and dimethyl-bicyclooctanes; tetrahydrobinor-S, pinane, camphane, decalin and alkyl decalins such as 1-methyldecalin; and 1,4,6,9-dimethanodecalin; bicyclo [4.3.2] undecane, bicyclo [5.3.0] decane, bicyclo [2.2.1] octane, tricyclo [5.2.1.0$^{2,6}$] decane, and the like; or any mixtures thereof. Hereto atom perfluoro compounds include perfluoro ethers such as $F$ - 2 - butyltetrahydrofuran, $F$ - 2 - butylfuran, $F$ - hydrofuran, the 1,2,2,2 - tetrafluoromethyl ether of $F$ - (2,5,8 - trimethyl - 3,6,9 - trioxa - 1 - dodecanol) and $F$ - n - methyl - morphiline. Acylic aliphatic compounds include $F$ - n - heptane and homologs.

Certain of the fluorine atoms of the foregoing materials may be substituted by other halogen atoms such as bromine as in perfluorobromo compounds. Included examples of these compounds are monobrominated compounds such as 1 - bromo - pentadecafluoro - 4 - isopropylcyclohexane, 1 - bromotridecafluoro - hexane, 1 - bromo - pentadecafluorooctane and 1 - bromo - pentadecafluoro - 3 - isopropylcyclopentane and perfluoro - 1 - bromobutylisopropyl ether, or polybrominated derivatives thereof.

As indicated above, the invention is applicable to fluorocarbons in the liquid or vapor state. However, solid compounds are treatable in accordance with the invention by dissolving in a suitable solvent or by melting or vaporization. Typical of such solid compounds are perfluoroadamantane and perfluoro-dimethyladamantane with relatively low levels of hydrogen contamination. These compounds may be dissolved in liquid perfluorocarbons (or other solvent), the purification of which may also be desired, and the resulting solution then treated. Suitable liquid fluorocarbons useful as solvents are $F$ - decalin, $F$ - tricyclo [5.2.1.0$^{2,6}$] decane, $F$ - menthane, $F$ - 1 - methyldecalin and $F$ - alkyladamantanes, including any mixtures thereof.

In the process of the invention, mixtures comprising perfluorocarbons and other fluorocarbons containing hydrogen in the molecule are treated with a base of an alkali or alkaline earth metal. The base contains not more than about 30 wt.% of water, and reacts with such other compounds to convert the latter to reaction products which are separated from unreacted perfluorocarbons. The toxicity and/or the amount of compounds containing hydrogen is reduced.

The method of the invention is conveniently practiced by adding the base to the fluorocarbon to be purified in a reaction vessel (preferably glass or glass-lined) equipped with an agitator, and heating the mixture with agitation to an elevated temperature sufficient to react the base with components of the fluorocarbon mixture. Preferably, the temperature is in the range from about 165°C to about 250°C. At temperatures at or above 250°C, some decomposition of the perfluorocarbons may take place, particularly with metal reactors, so such temperatures are not preferred. The reaction is maintained at the selected temperature for several minutes to several hours, e.g., about 1—5 hours. A typical temperature is about 170—180°C and a typical reaction time at this temperature is about 1—2 hours. However, both temperature and reaction time will depend on the specific fluorocarbon and the strong base. Since fluorocarbons for use in intravenous applications have relatively low vapor pressures and high boiling points, reflux temperature is often sufficient. Lower boiling fluorocarbons can be equivalently purified by heating the reaction mixture under pressure. Pressure reactors may be useful if treatment of a fluorocarbon in the vapor state is desired, or pressure can be used to permit higher reaction temperature by elevating the boiling points of the liquids.

The foregoing reaction conditions are effective for a strong base such as KOH containing about 15

4

wt.% water and a reaction mixture containing about 15—25 wt.% of such base, based on fluorocarbons containing about 95 ppm or less of hydrogen. A higher concentration of a base such as KOH may be used for purification of such fluorocarbons, but not appreciable advantage is obtained. As indicated, the reaction conditions will, of course, be modified to fit other fluorocarbons, bases containing other amounts of water or other solvent, and other proportions of reactants. The skilled practitioner can make such modifications without undue effort. Generally, if the fluorocarbon to be purified has a higher proportion of hydrogen-containing material, more vigorous reaction conditions, and/or higher proportions of base to fluorocarbon will be used or the reaction may be repeated. Elevated temperatures normally are required to obtain a desirable rate and degree of reaction.

During the reaction the purification can be followed by separating treated PFC from the reaction mixture in the manner described below, and then testing the PFC for color with diethylamine (DEA) or for hydrogen or olefin by infrared. Perfluorocarbons are colorless in the presence of DEA but are dark yellow, brown or black if hydrogen is present.

At the end of the reaction the mixture is cooled and water is added to dissolve any unreacted base, metal fluoride, and the highly oxygenated carbonaceous residue normally formed during the reaction. The addition of water causes the reaction mixture to form a top aqueous phase containing reaction products such as metal fluoride and degradation products, and a bottom fluorocarbon phase containing the substantially purified fluorocarbon. The phases may be separated by any suitable liquid/liquid separation method such as decanting or the use of separatory funnels.

For medical applications a further final purification of the fluorocarbon may still be desirable for removal of trace amounts of reaction products. Such traces sometimes impart a haze to the product but may in any event be detected by analysis as noted above.

In the case of treatment with the organic metal oxides these traces of reaction product can be extracted by washing the fluorocarbon with a ketone after which any trace of solvent is washed out with water. This extraction with solvent is more effective when each R group of the organic metal oxide used contained from about 4 to 10 or more carbon atoms and this is one reason that such a range was described earlier as preferred.

A typical additional purification procedure, when KOH or other inorganic oxide was used is to react the residual impurities in the fluorocarbon with an organic carbonyl compound such as a ketone (e.g. acetone). From the resulting two-phase system the PFC phase is separated from the organic phase and is then washed with a water miscible $C_1$—$C_8$ alkanol (e.g., methanol, ethanol) acidified with an organic acid such as those described above. An acid/alkanol ratio of about 1:9 by weight is suitable but other ratios can also be used, e.g., about 1:50 to 1:1. A final wash with water is used to remove the traces of acid and alkanol remaining in the purified fluorocarbon.

The material resulting from the final purifications as described above will, if the starting PGC material contained not over about 200 ppm hydrogen, contain less than about 25 ppm hydrogen by infrared, usually less than 5, and will be essentially nontoxic as determined by the vitro toxicity tests (such as the L-cell test described below) and/or conventional intravenous $LD_{50}$ testing in mice.

The following examples are intended as further illustration of the invention without limiting the scope thereof, except as set forth in the appended claims.

Example 1
Part A
4000 units by weight of a reaction mixture containing primarily perfluoro dimethyladamantane (F-DMA) and perfluoro trimethylbicyclononane (F-TMBCN), obtained by two pass, direct fluorination of DMA over cobalt trifluoride, and containing 95 ppm hydrogen is placed in a 5 liter, glass reaction flask equipped with a stirrer and a condenser vented to the atmosphere. The F-TMBCN was obtained as a result of ring-opening reactions occurring during the fluorination. 1000 units of 85% KOH (15% w/w $H_2O$) in pellet form are then added to the flask and stirring is commenced. Heat is applied to raise the temperature of the reaction mixture to reflux. The process takes about one hour to reach a final temperature of 170—175°C. During this heating stage, the contents of the flask change in appearance: the pellets break up as the reaction progresses and the color of the slurry changes from clear with white solids, to amber, and then dark brown or black. Some water may also form as droplets in the condenser. After reflux is reached, the mixture is held at these conditions for about two hours, at which time the heat is shut off and the mixture is cooled to approximately 90°C. 2000 units of water are then slowly added to the flask, and the temperature of the mixture rises. The temperature rise is due to heat of dilution of the unreacted KOH. After the water addition is complete, the mixture is agitated to fully dissolve the solids which have become encrusted on the walls of the reaction vessel. Separation of the water phase, containing substantially all of the reaction products, from the flourocarbon (PFC) phase is accomplished in a five liter separatory funnel. The two phases separate in about one hour. The fluorocarbon is then drained from the bottom of the separatory funnel and upon infrared analysis is found to contain less than 5 ppm hydrogen (limits of detection with the IR equipment used).

In an L-cell assay using Stock Littlefield strain L mouse fibroblasts as described by R. P. Geyer in his report for June 21, 1976 to June 20, 1978 under U.S. National Institutes of Health Contract No. NO1-HB-6-2926, pages IV-1 to IV-6, the fluorocarbon product exhibits 6.7% inhibition of cell growth (ICG) as

5

compared to the medium control (0% ICG). Medium control or assay control is medium plus cells dosed with additional medium equal in amount to the fluorocarbon in the test medium. Inhibition of cell growth theoretically ranges from 0 (no inhibition) to 100% (total kill) but 6.7% would generally be considered unsatisfactory in products intended for humans.

Part B

The fluorocarbon recovered in Part A above still has a slightly hazy appearance due to the presence of trace amounts of reaction product. Although the haze can be eliminated by conventional paper filtration, contact with the paper increases the toxicity (to 32.6% ICG) in the L-cell assay referred to in Part A above. Instead, the reaction product causing the haze is removed by treating the fluorocarbon phase in the following manner: 950 units of acetone is shaken in a separatory funnel for 5 minutes with the approximately 3800 units of product recovered from Part A. The fluorocarbon that is separated from the acetone has lost its hazy appearance, and any residual acetone is subsequently removed by washing with 950 units of a solution of acetic acid in methanol (1:9 by weight). Finally the trace level of methanol remaining is removed by treatment with 950 units of water. Both of these latter treatments are done in a manner similar to the acetone treatment described above. The final fluorocarbon product is free from haze and is found by infrared analysis to contain less than 5 ppm by weight hydrogen (limits of detection).

Example 2

In this Example the fluorocarbon material was obtained similarly to the material in Example 1, except that in Example 2, the fluorination product was distilled to obtain a heart cut mixture richer in F-DMA, the weight ratio being about 3.5 F-DMA to 1 F-TMBCN. In addition the cytotoxicity test is the L-cell suspension culture assay, Protocol 745, of Hazelton Laboratories America, Inc., Vienna, Virginia as described below. In this test procedure the assay control is cell culture and medium without fluorocarbon, the negative control is a fluorcarbon material purified as described in Example 1, Parts A and B, and having a low cytotoxicity as established by this assay, and the positive control is an untreated sample of fluorocarbon having known high toxicity.

L-cell test procedure

The cell line is L-929, clone of strain L (CCL-1), the species of origin being the mouse, $C_3H/An$ (fibroblast). The cells are derived from the parenteral strain L established by W. R. Earle (J. National Cancer Institute 4:165, 1943). The source is American Type Culture Collection, ATCC L-929. The cells are maintained as frozen stocks in liquid nitrogen. Working stocks are maintained in suspension in modified Waymouth MB 752/1 Medium Complete (Waymouth MB 752/1 supplemented with 10% Bovine Serum, 1% Glutamine, 0.5% Gentamicin, 0.1% Pluronics, 0.03% Methylcellulose and 0.05% Darvan) at 37°C. in an atmosphere of 5% $CO_2$ and air, and passaged once a week. A test compound is tested neat at one passage level, and both positive and negative controls are also tested along with an assay control (cell culture and medium, no fluorocarbon). Test compound and control materials are tested in quadruplicate.

For dosing, 50 ml. conical tubes are inoculated with test and control materials. Approximately 1—1.2×10⁵ cells/ml in modified Waymouth's medium are added in 15 ml quantities to each conical tube and gently mixed. The caps are securely tightened and the tubes placed in a roller drum to permit complete rotation of the tube. The tubes are rotated at 37°C for three days.

After three days incubation, aliquots of the cell cultures are diluted and stained with trypan blue. Using a hemacytometer, the cells are counted under a microscope to determine quantity and viability. Scoring is by averaging cell counts for each set of four replicates. From these results, the cell multiplication factor is determined. Inhibition of cell growth of the test compound as compared to the assay control cells is calculated. Percent inhibition of cell growth (% ICG) within five percentage units of the negative control values indicates lack of toxicity as compared to the negative control.

Percent inhibition of cell growth (ICG) is calculated as:

$$\% \ ICG = 100 - \% \ Growth$$

where

$$\% \ Growth = \frac{Mean \ cell \ count \ of \ treated \ sample}{Mean \ cell \ count \ of \ assay \ control} \times 100$$

Similarly to Example 1, the fluorocarbon test material, after treatment in Part A, is contacted with paper, resulting in 51% ICG. The product is then treated as in Part B, resulting in −8% ICG (an actual increase in cell growth). Apparently, either the paper was contaminated with toxic material which then transferred to the sample, or the cellulose of the paper reacted in some manner to introduce toxicity into the Part A product. However, the Part B treatment removed the toxic components. The foregoing and other results are set forth in Table I below from which it can be seen that the test material showed essentially the same lack of cytotoxicity as the negative and assay controls (no significance is attached to negative ICGs).

# EP 0 151 697 B1

## TABLE I

| Sample | Dose per 15 ml of media | ICG % |
|---|---|---|
| Assay control | 1.5 ml | 0 |
| Negative control | 1.5 ml | −5 |
| Positive control | 0.75 ml | 100 |
| Test material | 1.5 ml | −8 |

Assay control:
 Cell culture and medium without flourocarbon.
Negative control:
 Cell culture and medium with fluorocarbon previously purified as in Example 1, Parts A and B.
Positive control:
 Cell culture and medium with untreated sample of fluorocarbon.
Test Material:
 Cell culture and medium with fluorocarbon purified as in Example 2, Parts A and B.

Example 3

A fluorocarbon material (PFC) purified as in Example 1, Parts A and B, and then emulsified is differentially tested for acute toxicity in mice ($LD_{50}$, Bioassay Systems Corp., Boston, Massachusetts) with the results set forth in Table II. The fluorocarbon material was obtained similarly to the material in Example 2. It will be noted from the test results from Compositions C and D that the purified perfluorocarbon contributed no toxicity.

## TABLE II

| | | Aqueous[1] Composition (% w/v) | $LD_{50}(ml/kg)$[2] |
|---|---|---|---|
| (A) | NaCl | 0.9 | ≧229 |
| (B) | NaCl | 0.9 | |
| | Glycerol | 1.0 | 138—229 |
| (C) | NaCl | 0.9 | |
| | Glycerol | 1.0 | |
| | Pluronic F-68 surfactant[3] | 3.5 | 118.4 |
| (D) | NaCl | 0.9 | |
| | Glycerol | 1.0 | |
| | Pluronic F-68 surfactant | 3.5 | |
| | PFC | 20.0 | 118.4 |

[1]Sterile water
[2]95% confidence level, as practiced by Bioassay Systems Corp., Boston Massachusetts
[3]Polyoxyethylene-polyoxypropylene copolymer, molecular weight about 8200.

Example 4 (comparative)

A mixture of *F*-trimethylbicyclononane and *F*-dimethyladamantane, obtained as in Example 1 except using one-pass instead of two-pass fluorination, and therefore containing relatively high residual hydrogen (over 100 ppm), is treated ten times with 26.7% (w/w) KOH in water 105—108°C, and is then extracted ten times at room temperature with 5% KOH solution. This is a prior art procedure. Table III below gives the L-cell toxicity results (Hazelton procedure as in Example 2) wherein the negative, positive and assay controls are the same as before (Table I). It will be seen that even multiple treatments with the 26.7% KOH, as representative of prior purification techniques, resulted in a still toxic material (55% ICG), whereas material treated in accordance with the invention (negative control) shows no cytotoxicity.

7

TABLE III

| Sample | Dose per 15 ml of media | ICG % |
|---|---|---|
| Assay control | 1.5 ml | 0 |
| Negative control | 1.5 ml | −1 |
| Positive control | 0.75 | 94 |
| Test material | 1.5 | 55 |

Assay control:
   Cell culture and medium without fluorocarbon.
Negative control:
   Cell culture and medium with fluorocarbon purified in Example 1, Parts A and B.
Positive control:
   Cell culture and medium with untreated sample of fluorocarbon.
Test Material:
   Cell culture and medium with fluorocarbon purified as in Example 4.

Example 5
   The process of Example 1, Part A, is repeated in all essential respects except that the mixture of perfluoro-dimethyl adamantane and perfluoro-trimethylbicyclononane is refluxed for 15 minutes instead of 2 hours. The hydrogen level of the product is 8—10 ppm. This example shows that best results are obtained by extended refluxing, of the order of 1—2 hours or more, as in Part A of Example 1.

Example 6
   This example is another illustration of treatment of higher hydrogen content material.
   (A) 100 units by weight of perfluoro dimethyladamantane/perfluoro trimethylbicyclononane mixture, in a DMA:TMBCN weight ratio of approximately 2.6:1 and containing 401 ppm of hydrogen is treated with 50 units of 85% KOH (pellets) using two batches of 25 units each to keep the reaction from becoming thermally uncontrollable. The first 25 units of KOH is added to the fluorocarbon and the temperature of the mixture is gradually raised to 131°C. The second aliquot of 25 units of the 85% KOH is then added to the reaction mixture and the mixture is brought to reflux at 155°C. The intermediate product (I) of the reaction contains approximately 100 ppm hydrogen.
   (B) 75 units of intermediate product (I) is treated with 37 units of 85% KOh (pellets) at reflux (174°C) for two hours. The intermediate product (II) of the reaction contains 46 ppm hydrogen.
   (C) 67 units of intermediate product (II) is treated with 33 units of 85% KOH (pellets) at reflux (174°C) for two hours. The final product obtained is treated as in Part B of Example 1 and is found to contain less than 5 ppm hydrogen.

Example 7
   This example illustrates the effect of changing the KOH concentration. A mixture of perfluoro dimethyladamantane and perfluoro trimethylbicyclononane is distilled to obtain a heart cut mixture richer in perfluoro dimethyladamantane (3.5 to 1, perfluoro dimethyladamantane to perfluoro trimethylbicyclononane). The heart cut contains 52 ppm hydrogen. One aliquot (50 weight units) of this material is treated with 17.6 units of slightly pasty KOH (70% KOH, balance water) at between 196°C and 204°C for two hours in a pressurized reactor having a Teflon (trademark) liner. The product contains between 8 and 10 ppm hydrogen. A second aliquot (50 units) of the heart cut is treated with 25 units of syrupy KOH (50% KOH, balance water) at between 196 and 202°C for two hours in the same lined pressurized reactor. The hydrogen level in the latter product actually increases, to approximately 76 ppm. This indicates that a KOH strength of at least about 70% is required for efficient hydrogen removal.

Example 8
   A crude mixture of perfluoro dimethyladamantane and perfluoro trimethylbicyclonane, similar to Example 1 and containing 64 ppm hydrogen is treated with NaOH (98.7 wt.%) pellets in three runs (50 wt.%, 25 wt.% and 12.5 wt.% of the pellets based on the crude fluorocarbon mixture) substantially as described in Part A of Example 1 except for a reflux temperature of about 165°C. After addition of water to the reaction mixture, the fluorocarbon phase is separated. The product of each run is found to contain less than 5 ppm hydrogen (limits of detection).

# EP 0 151 697 B1

## Claims

1. A method of purifying a fluorocarbon mixture comprising perfluorocarbons and other fluorocarbons, characterized by contacting said mixture with a treating agent consisting essentially of a base of a metal selected from alkali metal and alkaline earth metal and containing not more than about 30 wt.% water, at a temperature sufficient to react such other fluorocarbons with said base, thereby to convert such other fluorocarbons to reaction products, and separating said reaction products from unreacted perfluorocarbons provided that when said base material is potassium hydroxide, diisobutylamine is absent.

2. A method according to claim 1, characterized in that said mixture comprises perfluorocarbons containing 7 to 18 carbon atoms and other fluorocarbons containing hydrogen in the molecule.

3. A method according to either of the preceding claims, characterized in that said base is an alkali metal hydroxide, alkoxide or aryloxide.

4. A method according to claim 3, characterized in that said base is potassium hydroxide.

5. A method according to claim 4, characterized in that said potassium hydroxide is in pellet form and wherein the amount of said potassium hydroxide in pellet form is at least 15 weight % based on the fluorocarbons.

6. A method according to claim 4, characterized in that said potassium hydroxide is slurried in said fluorocarbon-containing mixture.

7. A method according to any one of the preceding claims, characterized in that said flurocarbons are in a liquid state.

8. A method according to any one of the preceding claims, characterized in that the contacting is effected at a temperature in the range of 165 to 250°C.

9. A method according to any one of the preceding claims, characterized in that the reaction products are separated by adding water to dissolve unreacted base and any metal fluoride formed, and the two phases which form are separated into a fluorocarbon phase and an aqueous phase.

10. A method according to claim 9, characterized in that the fluorocarbon phase is further purified by reaction with an organic carbonyl compound followed by washing with an organic solvent, the purified product is separated from the organic solvent mixture, and the product is washed with water to remove the solvent.

11. A method according to claim 10, characterized in that said carbonyl compound is acetone and said organic solvent is a solution of acetic acid in methanol.

## Patentansprüche

1. Verfahren zum Reinigen eines Fluorkohlenstoffgemisches, das Perfluorkohlenstoffe und andere Fluorkohlenstoffe enthält, dadurch gekennzeichnet, daß das Gemisch mit einem Behandlungsmittel in Berührung gebracht wird, das im wesentlichen aus einer Base eines Metalls besteht, das aus den Alkalimetallen und den Erdalkalimetallen ausgewählt ist, und nicht mehr als etwa 30 Gew.% Wasser enthält, wobei die Berührung bei einer Temperatur herbeigeführt wird, die für eine Umsetzung der genannten anderen Fluorkohlenstoffe mit der Base genügt, so daß diese anderen Fluorkohlenstoffe in Reaktionsprodukte umgewandelt werden, die dann von nicht umgesetzten Perfluorokohlenstoffen getrennt werden, und wobei kein Butylamin vorhanden ist, wenn die Base Kaliumhydroxid ist.

2. Verfahren nach Anspruch 1, dadurch gekennzeichnet, daß das Gemisch Perfluorkohlenstoffe mit 7 bis 18 Kohlenstoffen enthält, sowie andere Fluorkohlenstoffe, die im Molekül Wasserstoff enthalten.

3. Verfahren nach einem der vorhergehenden Ansprüche, dadurch gekennzeichnet, daß die Base ein Alkalimetallhydroxid, -alkoxid oder -aryloxid ist.

4. Verfahren nach Anspruch 3, dadurch gekennzeichnet, daß die Base Kaliumhydroxid ist.

5. Verfahren nach Anspruch 4, dadurch gekennzeichnet, daß das Kaliumhydroxid in Pelletform vorliegt und die Menge des in Pelletform vorliegenden Kaliumhydroxids mindestens 15 Gew.% der Fluorkohlenstoffe beträgt.

6. Verfahren nach Anspruch 4, dadurch gekennzeichnet, daß das Kaliumhydroxid in dem Fluorkohlenstoff enthaltenden Gemisch aufgeschlämmt wird.

7. Verfahren nach einem der vorhergehenden Ansprüche, dadurch gekennzeichnet, daß sich die Fluorkohlenstoffe im flüssigen Zustand befinden.

8. Verfahren nach einem der vorhergehenden Ansprüche, dadurch gekennzeichnet, daß die Berührung bei einer Temperatur im Bereich von 165 bis 250°C herbeigeführt wird.

9. Verfahren nach einem der vorhergehenden Ansprüche, dadurch gekennzeichnet, daß zum Abtrennen der Reaktionsprodukte Wasser zum Auflösen von nicht umgesetzter Base und etwa gebildetem Metallfluorid zugesetzt wird und daß die beiden sich bildenden Phasen in einen Fluorkohlenstoffphase und eine wäßrige Phase getrennt werden.

10. Verfahren nach Anspruch 9, dadurch gekennzeichnet, daß zum weiteren Reinigen der Fluorkohlenstoffphase diese mit einer organischen Carbonylverbindung umgesetzt und danach mit einem organischen Lösungsmittel gewaschen wird, worauf das gereinigte Produkt von dem organischen Lösungsmittelgemisch abgetrennt und das Produkt zum Entfernen des Lösungsmittels mit Wasser gewaschen wird.

9

11. Verfahren nach Anspruch 10, dadurch gekennzeichnet, daß die Carbonylverbindung Aceton ist und daß das organische Lösungsmittel eine Lösung von Essigsäure in Methanol ist.

**Revendications**

1. Procédé de purification d'un mélange d'hydrocarbures fluorés comprenant des hydrocarbures perfluorées et d'autres hydrocarbures fluorés, caractérisé en ce que l'on met en contact ce mélange avec un agent de traitement essentiellement constitué par une base d'un métal choisi parmi un métal alcalin et un métal alcalino-terreux et ne contenant pas plus d'environ 30% en poids d'eau, à une température suffisante pour que lesdits autres hydrocarbures fluorés réagissent avec ladite base, pour transformer lesdits autres hydrocarbures fluorés en produits de réaction, et en ce que l'on sépare lesdits produits de réaction d'avec les hydrocarbures perfluorés n'ayant pas réagi, à condition que, lorsque cette substance basique est l'hydroxyde de potassium, de la diisobutylamine soit absente.

2. Procédé selon la revendication 1, caractérisé en ce que ledit mélange comprend des hydrocarbures perfluorés contenant 3 à 18 atomes de carbone et d'autres hydrocarbures fluorés contenant de l'hydrogène dans la molécule.

3. Procédé selon l'une quelconque des revendications précédentes, caractérisé en ce que ladite base est un hydroxyde, un alcoxyde ou un aryloxyde de métal alcalin.

4. Procédé selon la revendication 3, caractérisé en ce que ladite base est l'hydroxyde de potassium.

5. Procédé selon la revendication 4, caractérisé en ce que l'hydroxyde de potassium se présente sous forme de pastilles et en ce que la quantité de l'hydroxyde de potassium sous forme de pastilles est d'au moins 15% en poids, par rapport aux hydrocarbures fluorés.

6. Procédé selon la revendication 4, caractérisé en ce que l'hydroxyde de potassium est mis en suspension dans le mélange contenant les hydrocarbures fluorés.

7. Procédé selon l'une quelconque des revendications précédentes, caractérisé en ce que les hydrocarbures fluorés sont à l'état liquide.

8. Procédé selon l'une quelconque des revendications précédentes, caractérisé en ce que la mise en contact s'effectue à une température dans l'intervalle de 165 à 250°C.

9. Procédé selon l'une quelconque des revendications précédentes, caractérisé en ce que les produits de réaction sont séparés par addition d'eau pour dissoudre la base n'ayant pas réagi et tout fluorure métallique formé, et les deux phases qui se forment sont séparées en une phase d'hydrocarbures fluorés et une phase aqueuse.

10. Procédé selon la revendication 9, caractérisé en ce que la phase d'hydrocarbures fluorés est encore purifiée par réaction avec un composé carbonyle organique, suivie d'un lavage avec un solvent organique, le produit purifié est séparé du mélange de solvunts organiques et le produit est lavé avec de l'eau pour éliminer le solvant.

11. Procédé selon la revendication 10, caractérisé en ce que le composé carbonyle est l'acétone et le solvant organique est une solution d'acide acétique dans le méthanol.